**Europäisches Patentamt**

**European Patent Office**

**Office européen des brevets**

(19)

(11) Veröffentlichungsnummer : **0 320 441 B1**

# (12) EUROPÄISCHE PATENTSCHRIFT

(45) Veröffentlichungstag der Patentschrift :
28.08.91 Patentblatt 91/35

(51) Int. Cl.$^5$ : **A61F 2/06**

(21) Anmeldenummer : **88810731.5**

(22) Anmeldetag : **26.10.88**

(54) **Verfahren und Anlage zum Konditionieren von mit lebenden Zellen beschichteten Kunststoffträgern.**

(30) Priorität : **07.12.87 CH 4768/87**

(43) Veröffentlichungstag der Anmeldung :
**14.06.89 Patentblatt 89/24**

(45) Bekanntmachung des Hinweises auf die
Patenterteilung :
**28.08.91 Patentblatt 91/35**

(84) Benannte Vertragsstaaten :
**AT DE FR GB IT**

(56) Entgegenhaltungen :
**WO-A-82/03764**
**FR-A- 2 290 182**

(73) Patentinhaber : **GEBRÜDER SULZER**
**AKTIENGESELLSCHAFT**
**Zürcherstrasse 9**
**CH-8401 Winterthur (CH)**

(72) Erfinder : **Müller, Werner, Dr.**
**Wannenstrasse 6**
**CH-8542 Wiesendangen (CH)**
Erfinder : **Hensel, Marie-Claude**
**Breitistrasse 62**
**CH-8614 Bertischikon (CH)**
Erfinder : **Huber, August**
**Gotthelfstrasse 11**
**W-8352 Räterschen (DE)**

EP 0 320 441 B1

## Beschreibung

Die Erfindung betrifft ein Verfahren zum Konditionieren von mit lebenden Zellen beschichteten Kunststoff-Trägern, die als Ersatz für eine Organ- oder Gefässwand im menschlichen Körper dienen und aus einer wässrigen Suspension auf einer Seite mit epithelialen Zellen beschichtet worden sind.

Es ist (z.B. aus der WO 82/03764 oder der FR-A-2290182) bekannt, als Gefässe, insbesondere als Blutgefässe dienende künstliche Träger, z.B. Membranen aus Polyurethan oder anderen geeigneten Elastomer-Kunststoffen, mit epithelialen, z.B. Endothel, -Zellen zu beschichten ; ein Verfahren für solche Beschichtungen besteht z.B. darin (siehe WO 82/03764), die zuvor einem Patienten entnommenen, gegebenenfalls in einer Kultur vermehrten Zellen auf die Träger durch Sedimentieren aus einer wässrigen Suspension aufzubringen. Blutgefässähnliche, rohrförmige Träger oder Membranen werden dabei in gestrecktem Zustand auf einer drehbaren Vorrichtung gehalten und mit einer Suspension der Zellen gefüllt. In bestimmten Zeitabschnitten — die sich beispielsweise von einigen Sekunden auf einige Minuten steigern — werden die zu beschichtenden Träger um einen bestimmten Winkel, der einen Bruchteil eines Vollkreises beträgt, gedreht und die Zellen aufgrund der Schwerkraft aus der Suspension auf dem Träger abgelagert, bevor eine weitere Drehung erfolgt, um eine bestimmte primäre Verteilung der Zellen auf dem Träger zu erhalten.

In der Praxis hat sich gezeigt, dass die Haftung der auf diese Weise auf der künstlichen Gefässwand abgelagerten Zellen, besonders wenn sie Scherkräften eines über sie hinwegströmenden Blutstromes ausgesetzt werden, ungenügend ist. Aufgabe der vorliegenden Erfindung ist es daher, die Haftung von Zellen auf der künstlichen Gefässwand zu verbessern.

Diese Aufgabe wird dadurch gelöst, dass die mit den Zellen beschichtete Seite des Trägers einem pulsierenden Fluss einer Nährlösung für die Zellen ausgesetzt wird, dessen quasiphysiologische Pulsationen kurzzeitige Umkehrungen der Flussrichtung bewirken, wobei nach dem Beschichten während einiger Stunden zunächst die Nährlösung nahe der zellenbeschichteten Seite des Trägers in regelmässigen Zeitabständen durch Flussimpulse erneuert wird, und anschliessend die Fluss- und die Pulsationsgeschwindigkeit über Tage von Null auf einen mit den physiologischen Werten natürlicher Gefässe vergleichbaren Maximalwert gesteigert werden ; eine Anlage zur Durchführung des Verfahrens ist gekennzeichnet durch ein in sich geschlossenes, mit Nährlösung gefülltes Schlauchleitungssystem, in dem mindestens eine Pumpe, ein Gasaustauscher, eine Behandlungskammer für den zu konditionierenden Träger, ein einstellbares Drosselorgan und ein Windkessel vorhanden sind.

Durch die allmähliche Steigerung der Pulse pro Zeiteinheit, sowie von Strömungsgeschwindigkeit und dynamischem Druck in der an dem mit Zellen belegten Träger vorbeifliessenden Nährlösung bis auf Werte ähnlich denjenigen, die in einer Arterie vorhanden sind, werden die abgelagerten Zellen an die Scherkräfte "gewöhnt", die bei einer Implantation in dem Blutkreislauf eines Menschen auf sie einwirken. Von entscheidender Bedeutung ist bei dem neuen Verfahren, dass diese Gewöhnung (Konditionierung) allmählich über Zeiträume von Tagen mit stufenweise oder kontinuierlich gesteigerten Flussgeschwindigkeiten erfolgt.

Mit Vorteil wird die Konditionierung des mit Zellen belegten Trägers bei einem statischen Druck von 0,07 bis 0,2 bar (50 bis 150 mm Hg) durchgeführt, einem Druckbereich, ähnlich den in einem natürlichen arteriellen Gefäss herrschenden Druckverhältnissen. Der Aufwand an Nährlösungsmenge lässt sich mit Vorteil reduzieren, wenn die Nährlösung bei der Konditionierung des Trägers in einem Kreislauf geführt wird, in dem sie einen Gasaustauscher durchströmen, um Gas- und pH-Bedingungen der Nährlösung zu kontrollieren.

Die einstellbare Drosselung des Nährlösungsflusses, in Strömungsrichtung nach dem zellenbeschichten Träger, hat zum Ziel, dass ein die physiologischen Verhältnisse simulierender "Rückfluss", auftritt ; die Geschwindigkeit dieses Rückflusses kann in gewissen Grenzen variiert werden, wobei der Rückfluss mit stärker werdender Drosselung ansteigt.

Damit die bei den Flusspulsationen entstehenden dynamischen Drücke physiologisch sinnvolle Druckwerte und Druckgradienten nicht überschreiten, ist es zweckmässig, die bei den Pulsationen erregten dynamischen Druckamplituden in der Nährlösung so zu dämpfen, dass bei diesen Drücken die Druckgradienten Werte von etwa 2,6 bar/sec (2000 mm Hg/sec) nicht überschreiten. Weiterhin kann es für die Konditionierung des zellbeschichten Trägers vorteilhaft sein, wenn dieser während der Konditionierung um eine zu seiner Längsachse parallele Achse in einem Vollkreis hin und her geschwenkt wird, wobei das Durchlaufen eines Vollkreises beispielsweise in 1 bis 20 Minuten erfolgt.

Eine einfache Massnahme zur Einstellung eines bestimmten statischen Druckes in dem Schlauchleitungssystem der Anlage, insbesondere in dem zellbeschichten Trägerstück, kann darin bestehen, dass die statische Höhe des höchsten Punktes des Schlauchleitungssystem relativ zur Behandlungskammer einstellbar ist.

Eine vorteilhafte Konstruktion für die Behandlungskammer ergibt sich, wenn diese mit Durchflussöffnungen aufweisenden Halterungen für die zu konditionierende Länge eines schlauchförmigen Trägers und mit zwei Anschlüssen für das Schlauchleitungssystem versehen ist, von denen einer an ein Ende einer Trägerhalterung und der andere in den den Träger umgebenden Innenraum der Behandlungskammer führen ; die Behandlungskammer kann darüberhinaus mit einer Schwenkvorrichtung versehen sein, durch die sie um ihre Längsmittelachse auf einem Vollkreis geschwenkt werden kann.

Für die Erzeugung der Flusspulsationen wird vorteilhafterweise aus Sterilisationsgründen eine Schlauchquetschpumpe verwendet, die mit zwei einander diametral gegenüberliegenden umlaufenden Quetschrollen versehen ist, zwischen denen eine geschlossene Schleife des Schlauchleitungssystems angeordnet ist, deren Länge grösser ist als der Bogen des von ihnen erzeugten halben Bahnkreises.

Für eine Reduzierung des auf den zellenbeschichteten Gefässträger einwirkenden, durch die Pumpe erzeugten dynamischen Druckes ist es vorteilhaft, wenn die zwischen dem jeweiligen Schlaucheintritt und - austritt in den bzw. aus dem Bahnkreis liegende Sehne für die Schleife grösser ist als für die Schlauchleitungen. Dadurch erfolgt das Oeffnen und Schliessen eines Durchflusses durch die Schlauchschleife der Pumpe in "Rückwärtsrichtung", d.h. entgegen der Strömungsrichtung der Nährlösung zeitlich verschieden von dem Oeffnen und Schliessen des Durchflusses in Vorwärtsrichtung, d.h. in Richtung auf den zellenbeschichteten Träger. Damit kann ein Teil des Druckes in der Schlauchschleife nach rückwärts abgebaut werden, ehe der Vorwärtsdurchfluss beendet ist.

Im folgenden wird die Erfindung anhand eines Ausführungsbeispiels im Zusammenhang mit der Zeichnung näher erläutert.

Fig. 1    gibt den schematischen Aufbau einer Anlage zur Durchführung des neuen Verfahrens wieder ;

Fig. 2    ist eine schematische Darstellung einer Schlauchquetschpumpe ;

Fig. 3    zeigt in einem schematischen Diagramm ein Beispiel für die Flussgeschwindigkeit der Nährlösung, vorbei an dem zellenbeschichteten Träger in Abhängigkeit von der Zeit, wobei als Ordinate der Fluss in Prozenten des bekannten physiologischen Blutstromes angegeben ist.

In einem Thermostat 1, dessen Temperatur konstant auf etwa 37°C gehalten wird, ist ein einen geschlossenen Kreislauf bildendes Schlauchleitungssystem 2 untergebracht, dessen Schläuche aus einem bioinerten sterilisierbaren Material, beispielsweise aus Silikon, Polyvinylchlorid oder Polyurethan, bestehen. Die einzelnen Schläuche verbinden eine Behandlungskammer 3, die im weiteren noch näher beschrieben wird, einerseits über ein einstellbares Drosselorgan 4 mit einem Gasaustauscher 5 für den Austausch von Gasen, die im Fluss einer Nährlösung 7 gelöst sind, und andererseits mit einer ebenfalls noch näher zu beschreibenden Schlauchquetschpumpe 6, die in dem Kreislauf des Schlauchleitungssystems 2 den pulsierenden Fluss der Nährlösung 7 erzeugt und aufrechterhält. An der "Saugseite" der Pumpe 6 führt ein weiterer Schlauchabschnitt, in dem zur Dämpfung von Druckamplituden ein Windkessel 8 vorgesehen ist, zu einem einfachen T-Stück 9, das eine Einspeisestelle für frische und eine Absaugstelle für verbrauchte Nährlösung 7 bildet. Der Kreislauf ist durch eine Schlauchverbindung zwischen dem Gasaustauscher 5 und dem T-Stück 9 geschlossen, dessen freier "Arm" als Mutterkonus für entsprechende Gegenkonen von Injektionsspritzen ausgebildet und mit einer Verschlusskappe 10 steril verschlossen ist. Mit Hilfe des T-Stückes 9 kann, beispielsweise einmal pro Tag, ein Teil der "verbrauchten" Nährlösung 7 in dem Schlauchsystem 2 erneuert werden, wobei jeweils ein Teil oder das ganze Volumen der Nährlösung ersetzt werden.

Das obere Niveau 11 des Flüssigkeitsstandes im Schlauchleitungssystem 2 über der Behandlungskammer 3 ist variierbar, um in diese Kammer 3 einen bestimmten statischen Druck — von beispielsweise etwa 70 mbar bis 200 mbar (50 bis 150 mm Hg) zu erzeugen.

Der Gasaustauscher 5, der mit der umgebenden Atmosphäre über ein Sterilfilter 12 in Verbindung steht, enthält eine Spirale 13, auf der die Nährlösung, beispielsweise die im Handel erhältliche Lösung M199 — eine mit 20 Vol.% fötalem Rinderserum (FCS) als Quelle für Aminosäuren angereicherte 0.9 (massen-)%ige physiologische Kochsalzlösung — aufgrund der Schwerkraft abwärts fliesst. Als von der Nährlösung 7 aufzunehmendes Gas in Medizinalqualität wird dem Gasaustauscher 5 ein Gemisch von 3-5 Vol.% Kohlendioxid ($CO_2$) mit einem luftähnlichen Gas aus Sauerstoff ($O_2$) und Stickstoff ($N_2$) über einen Gaswäscher 14 zugeführt, in dem das genannte Gasgemisch, das Medizinalqualität hat, zur Sättigung mit Wasserdampf bidestilliertes Wasser 20 durchströmt. Das Gasgemisch wird ausserhalb der Thermostaten 1 einer Gasflasche 15 entnommen und über ein Druckreduzierventil 16 und einen Feindruckregler 17 einem Dosierelement 18, beispielsweise einem einstellbaren Drosselorgan, zugeführt, an das ein Strömungsmessgerät 19 anschliesst, das mit dem bereits innerhalb des Thermostaten 1 angeordneten Gaswäscher 14 über einen weiteren Sterilfilter 12 verbunden ist.

Der durch das Dosierelement 18 eingestellte Gasstrom beträgt einige ml/min, d.h. einige in dem destillierten Wasser 20 aufsteigende Gasblasen pro Sekunde, wobei im Gaswäscher 14 und Gasaustauscher 5 ein Gasdruck etwa 0,3 bar (225 mm Hg) aufrechterhalten wird. In dem Gastauscher 5 nimmt die Nährlösung 7 in erster Linie Sauerstoff auf und gibt dafür von den Zellen erzeugtes CO2 ab.

Die Behandlungskammer 3, in der als Träger eine Membran 23 schon während der nicht zur Erfindung gehörenden Beschichtung mit epithelialen Zellen gehalten und aufbewahrt ist, besteht aus einem durchsichtigen Gehäuse 21 aus bioinertem Material, z.B. Glas ; das Gehäuse 21 ist mit einer durch einen nicht dargestellten Antrieb angetriebenen und gesteuerten Schwenkvorrichtung 22 versehen, durch die es so hin und der geschwenkt werden kann, dass es bei jeder Bewegung einen Winkel von $2\pi$ überstreicht. Damit wird erreicht, dass die mit Endothel-Zellen beschichtete, schlauchförmige Träger 23 bei jeder Schwenkbewegung einmal um die Längsachse gedreht wird, so dass alle Bereiche seines Umfanges während einer solchen Schwenkbewegung, die kontinuierlich oder schrittweise erfolgen kann, einmal "unten" liegen.

Der Träger 23 ist in gestreckter Lage auf zwei mit Durchflussöffnungen 24 versehenen Halterungen 25 gehalten, von denen eine an einen Anschluss 26 des Schlauchleitungssystems 2 angeschlossen ist, während die Durchflussöffnung der anderen Halterung 25 in dem Innenraum 27 des Gehäuses 21 ausserhalb des schlauchförmigen Trägers 23 endet ; mit diesem Innenraum 27 steht das Schlauchleitungssystem 2 über den zweiten Anschluss 28 in Verbindung ; selbstverständlich kann dieser auch über eine Leitung mit dem anderen Ende der Membran 23 verbunden sein.

Die Schlauchquetschpumpe 6 umfasst ein Gehäuse 29 mit einem kreiszylindrischen Hohlraum 30, in dem von einem nicht dargestellten Motor angetrieben zwei einander diametral gegenüberliegende Quetschrollen 31 in Richtung des Pfeiles 38 umlaufen. Diese werden über Federn, deren Federkraft einstellbar ist, gegen die den Bahnkreis 32 der Quetschrollen 31 bildenden Mantelfläche des Hohlraumes 30 gepresst. Auf einem Teil des Umfanges dieses Bahnkreises 32 verläuft zwischen Rollen 31 und Kreis 32 eine Schleife 33 des Schlauchleitungssytems 2, an das die Pumpe über Anschlüsse 34 und 35 angeschlossen ist. Mit dem Quetschrollen 31 umlaufend sind zwei Führungsrollen 36 vorgesehen, durch die der in der Pumpe verlaufende Schlauch bei jedem Umlauf in den Hohlraum 30 zurückgeführt oder in ihm gehalten wird.

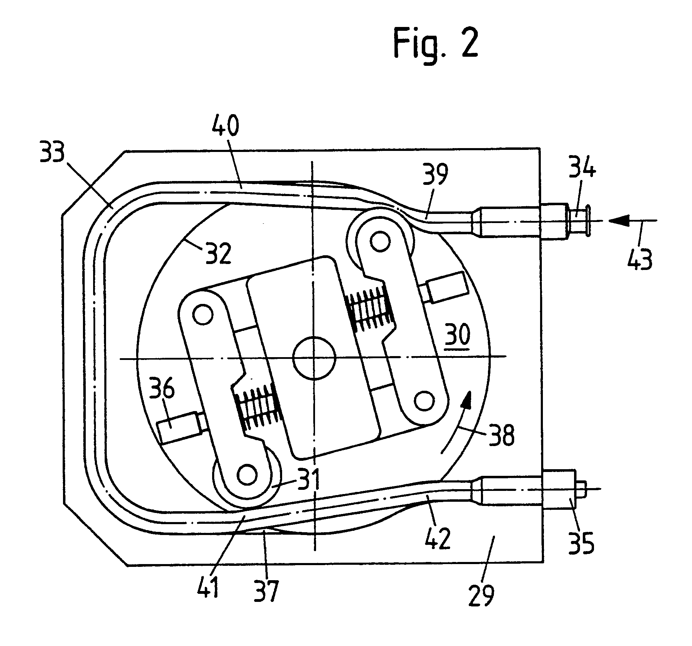

Wie Fig. 2 erkennen lässt, tritt das die Schleife 33 erzeugende Schlauchstück bei 39 in den Hohlraum 30 ein und verlässt diesen am Austritt 40 in tangentialer Richtung, wobei es in eine Ausnehmung 37 des Gehäuses 29 eingelegt ist. Die gegenüber dem Bahnkreis 32 verlängerte Schleife 33 tritt bei 41 tangential wieder in den Bahnkreis 32 ein, ehe sie diesen am Austritt 42 verlässt und am Anschluss 35 endet. Der Eintritt 39 und der Austritt 42 der Schlauchleitungen und der Eintritt 40 und der Austritt 41 der Schleife 33 in bzw. aus dem Bahnkreis 32 sind dabei zur konstruktiven Vereinfachung spiegelsymmetrisch zu einem in der Zeichnung horizontalen Durchmesser des Bahnkreises 32 bzw. des Hohlraumes 30 angeordnet, wobei der je eine Kreissehne dieses Hohlraumes 30 bildende Abstand zwischen dem Eintritt 40 und dem Austritt 41 der Schleife 33 grösser ist als derjenige zwischen dem Eintritt 39 und dem Austritt 42 des Schlauchleitungssystems 2.

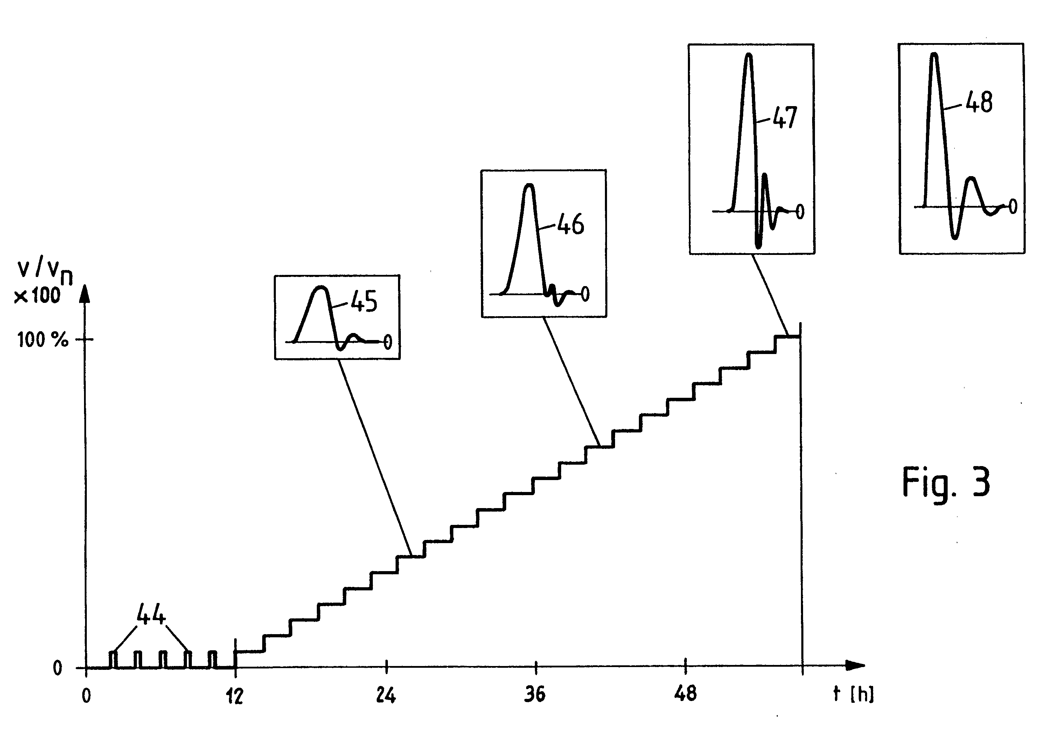

Durch die gegenüber dem Bahnkreis der Quetschrollen 31 verlängerte Schleife 33 werden bei einer Rotation der Quetschrollen 31, die zwischen 0 und 100 Umdrehungen pro Minute (U/min) betragen kann, einerseits die Pulsationen mit dem geforderten Rückfluss in der Nährflüssigkeit 7 erzeugt ; andererseits bewirken die relativ kurzen Wege zwischen dem Eintritt 39 und dem Austritt 40 bzw. dem Eintritt 41 und dem Austritt 42, während denen ein Quetschen des Schlauches durch die Rollen 31 erfolgt, daß die erzeugenden Flusspulsationen und mit ihnen die dynamischen Drücke die Form der in Fig. 3 gezeigten quasiphysiologischen Flussverläufe annehmen.

Die grössere Sehnenlänge für die Schleife 33 am Bahnkreis 32 gegenüber der entsprechenden Sehnenlänge für das Schlauchleitungssystem 2 hat zur Folge, dass beispielsweise — bei der durch die Pfeile 43 angedeuteten Strömungsrichtung der Nährlösung 7 durch die Pumpe 6 — die obere Quetschrolle 31 am Austritt 40 eine "Rückwärtsverbindung" der Schleife 33 bereits freigibt, ehe die untere Rolle 31 den Vorwärtsweg am Austritt 42 öffnet. Dadurch wird ein Teil des Druckes in der Schleife 33 zunächst nach rückwärts abgebaut und vom Windkessel 8 aufgenommen, ehe eine Druckspitze nach "vorn" in Richtung auf den zellenbeschichtete Träger 23 läuft. So können Drucküberlastungen dieser Träger 23 verhindert werden.

In dem Diagramm der Fig. 3 ist als Abszisse die Zeit t in Stunden (b) aufgetragen, während, wie erwähnt, die Ordinate die Flussgeschwindigkeiten v durch die Membran 23 wiedergibt, ausgedrückt in %-en der — vom Durchmesser der Schlauchmembran bzw. des natürlichen Gefässes abhängigen — bekannten physiologischen Flussgeschwindigkeit $v_n$ in einem natürlichen arteriellen Gefäss. Für ein Gefäss von 4 mm Durchmesser beträgt der Absolutwert der Spitzengeschwindigkeit dieses Flusses in der Regel beim gesunden Menschen nicht mehr als 1 m/sec.

Nachdem ein Träger 23, beispielsweise auf die eingangs beschriebene Weise, vollständig mit Zellen belegt worden ist, bleibt sie in der Behandlungskammer 3 bei Verwendung von vorkultivierten Zellen zunächst während etwa 12 Stunden mit ruhender Nährlösung 7 gefüllt ; durch kurze, einige Sekunden oder Minuten dauernde Einschaltimpulse 44 für die Pumpe 6 wird während dieser Zeit die in dem Träger 23 "stehende" Nährlösung 7 etwa alle 2 bis 6 Stunden erneuert. Während der Ruhezeit — in der die Kammer 3 die erwähnten Schwenkbewegungen auf einem Vollkreis gegebenenfalls bereits ausführt, die etwa eine Frequenz von 1 bis 20 Hin- und Herbewegungen in der Minute hat — breiten sich die Zellen auf der Membran 23 aus.

4

Nach 12 Stunden wird die Pumpe 6 im Dauerbetrieb in Bewegung gesetzt, wobei sie zunächst beispielsweise zwei Umdrehungen pro Minute ausführt, was — da zwei Quetschrollen 31 vorhanden sind — einer Pulsfrequenz des Flusses von 4 Pulsen/min entspricht. Die Umdrehungen der Pumpe 6 pro Minute können nun schrittweise oder kontinuierlich gesteigert werden bis etwa natürliche Pulsfrequenzen von 60 bis 100 Pulsen/min, also 30 bis 50 Umdrehungen pro Minute für die Pumpe 6 erreicht sind. Bei stufenweisem Steigern in Abständen von etwa zwei Stunden ergibt sich dabei der in Fig. 3 gezeigte Verlauf für die Flussgeschwindigkeit; selbstverständlich sind auch — linear oder nicht linear — kontinuierlich ansteigende Verläufe möglich.

Das in der Anlage dabei erzielte Flussprofil ist für drei verschiedene Werte der Flussgeschwindigkeit in den Kurvenverläufen 45 bis 47 der Fig. 3 wiedergegeben, während die Kurve 48 zum Vergleich den Fluss in einer natürlichen Arteria femoralis des Menschen schematisch zeigt. Für einen Fluss durch die Membran 23 von 100 % ist dabei die Amplitude der Kurve 47 an diejenige des natürlichen Gefässes angeglichen worden, da ihr Kurvenverlauf stark vom Durchmesser und der anatomischen Lage der Arterie abhängt.

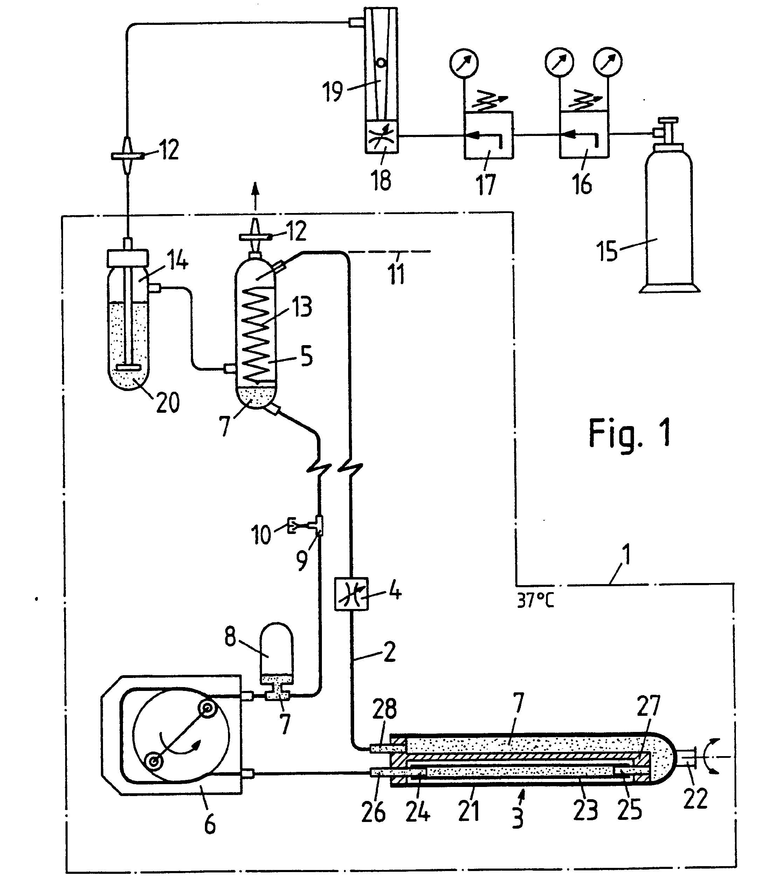

Das Nullniveau der Kurven 45 bis 47 liegt bei einem statischen Druck von 100 bis 105 mbar (70 bis 80 mm Hg) und wird in der Anlage mit Hilfe der Niveauhöhe 11 (Fig. 1) eingestellt.

Im vorliegenden Beispiels ist die quasiphysiologische Flussbelastung der beschichteten Membran 23 nach etwa 2 1/2 Tagen erreicht. Nach mindestens weiteren 24 Stunden ist die konditionierte Membran zur Implantation in den Blutkreislauf bereit. Im Tierversuch hat sich eine stark verbesserte Haftung der Zellen bei konditionierten Membranen 23 gegenüber solchen, die nicht nach dem neuen Verfahren behandelt worden sind, ergeben.

## Patentansprüche

1. Verfahren zum Konditionieren von mit lebenden Zellen beschichteten Kunststoff-Trägern, die als Ersatz für eine Organ- oder Gefässwand im menschlichen Körper dienen und aus einer wässrigen Suspension auf einer Seite mit epithelialen Zellen beschichtet worden sind, **dadurch gekennzeichnet, dass** die mit den Zellen beschichtete Seite der Träger (23) einem pulsierenden Fluss einer Nährlösung (7) für die Zellen ausgesetzt wird, dessen quasiphysiologische Pulsationen kurzzeitige Umkehrungen der Flussrichtung bewirken, wobei nach dem Beschichten während einiger Stunden zunächst die Nährlösung (7) nahe der zellenbeschichteten Seite der Träger (23) in regelmässigen Zeitabständen durch Flussimpulse erneuert wird, und anschliessend die Fluss- und die Pulsationsgeschwindigkeit über Tage von Null auf einen mit den physiologischen Werten natürlicher Gefässe vergleichbaren Maximalwert gesteigert werden.

2. Verfahren nach Anspruch 1, **dadurch gekennzeichnet, dass** die Konditionierung des Trägers (23) bei einem statischen Druck von 0,07 bis 0,2 bar (50 bis 150 mm Hg) durchgeführt wird.

3. Verfahren nach Anspruch 1 oder 2, **dadurch gekennzeichnet, dass** die Nährlösung (7) bei der Konditionierung des Trägers (23) in einem Kreislauf geführt wird, in dem sie einen Gasaustauscher (5) durchströmt.

4. Verfahren nach Anspruch 3, **dadurch gekennzeichnet, dass** der Nährlösungsfluss, in Strömungsrichtung nach dem Träger (23), einstellbar gedrosselt wird.

5. Verfahren nach Anspruch 3 oder 4, **dadurch gekennzeichnet, dass** bei den Pulsationen erregte dynamische Druckamplituden in der Nährlösung (7) gedämpft werden.

6. Verfahren nach einem der Ansprüche 1 bis 5, **dadurch gekennzeichnet, dass** der maximale Druckgradient des sich parallel zu dem Fluss aufbauenden dynamischen Druckes auf Werte kleiner etwa 2,6 bar/sec (2000 mm Hg/sec) begrenzt wird.

7. Verfahren nach einem der Ansprüche 1-6, **dadurch gekennzeichnet, dass** der zellenbeschichtete Träger (23) während der Konditionierung um eine zu seiner Längsachse parallele Achse auf einem Vollkreis hin und her geschwenkt wird.

8. Anlage zur Durchführung des Verfahrens nach einem der Ansprüche 1 bis 7, **gekennzeichnet durch** ein in sich geschlossenes, mit Nährlösung (7) gefülltes, Schlauchleitungssystem (2), in dem mindestens eine Pumpe (6), ein Gasaustauscher (5), eine Behandlungskammer (3) für den zu konditionierenden Träger (23), ein einstellbares Drosselorgan (4) und ein Windkessel (8) vorhanden sind.

9. Anlage nach Anspruch 8, **dadurch gekennzeichnet, dass** die statische Höhe des höchsten Punktes (11) des Leitungssystems (2) relativ zur Behandlungskammer (3) einstellbar ist.

10. Anlage nach Anspruch 8, **gekennzeichnet durch** eine Behandlungskammer (3) mit Durchflussöffnungen (24) aufweisenden Halterungen (25) für die zu konditionierende Länge eines schlauchförmigen Trägers (23) und mit zwei Anschlüssen (26, 28) für das Schlauchleitungssystem (2), von denen einer (26) an eine der Trägerhalterungen (25) und der andere (28) in den den Träger (23) umgebenden Innenraum (27) der Behandlungskammer (3) oder an das andere Ende des Trägers (23) führen.

11. Anlage nach Anspruch 10, **dadurch gekennzeichnet, dass** die Behandlungskammer (3) um ihre Längsmittelachse auf einem Vollkreis schwenkbar ist.

12. Anlage nach Anspruch 8, **gekennzeichnet durch** eine Schlauchquetschpumpe (6) mit zwei einander diametral gegenüberliegenden, umlaufenden Quetschrollen (31), zwischen denen eine geschlossene Schleife (33) des Schlauchleitungssystems (2) angeordnet ist, deren Länge grösser ist als der Bogen des von den Rollen (31) erzeugten halben Bahnkreises (32).

13. Anlage nach Anspruch 12, **dadurch gekennzeichnet, dass** die zwischen dem jeweiligen Schlaucheintritt (39, 41) und -austritt (40, 42) in den bzw. aus dem Bahnkreis (32) liegende Sehne für die Schleife (33) grösser ist als für die Schlauchleitungen.

## Claims

1. A process for conditioning plastic supports coated with living cells and intended as a replacement for the wall of an organ or vessel in the human body, and which have been coated with epithelial cells on one side from an aqueous suspension, characterised in that the side of the support (23) coated with the cells is subjected to a pulsating flow of a nutrient solution (7) for the cells, the quasi-physiological pulsations of which cause brief reversals of the direction of flow, and after the coating the nutrient solution (7) near the cell-coated side of the support (23) is first renewed at regular intervals for some hours by flow pulses and then the flow and pulsation speed are increased over a period of days from zero to a maximum value comparable to the physiological values of natural vessels.

2. A process according to claim 1, characterised in that the conditioning of the support (23) is carried out at a static pressure of 0.07 to 0.2 bar (50 to 150 mm Hg).

3. A process according to claim 1 or 2, characterised in that the nutrient solution (7) during conditioning of the support (23) is circulated in a cycle in which it flows through a gas exchanger (5).

4. A process according to claim 3, characterised in that the nutrient solution flow is adjustably restricted downstream of the support (23).

5. A process according to claim 3 or 4, characterised in that dynamic pressure amplitudes produced during the pulsation in the nutrient solution (7) are damped.

6. A process according to any one of claims 1 to 5, characterised in that the maximum pressure gradient of the dynamic pressure building up parallel to the flow is limited to values less than about 2.6 bar/sec (2000 mm Hg/sec).

7. A process according to any one of claims 1 to 6, characterised in that during the conditioning the cell-coated support (23) is rocked to and fro over a full circle about an axis parallel to its longitudinal axis.

· 8. An installation for performing the process according to any one of claims 1 to 7, characterised by a closed hosepipe system (2) filled with nutrient solution (7) and containing at least one pump (6), a gas exchanger (5), a treatment chamber (3) for the support (23) for conditioning, an adjustable restrictor means (4) and an air chamber (8).

9. An installation according to claim 8, characterised in that the static level of the highest point (11) of the hosepipe system (2) is adjustable relatively to the treatment chamber (3).

10. An installation according to claim 8, characterised by a treatment chamber (3) with holders (25) formed with passage apertures (24) for the length of a support hose (23) for conditioning, and having two connections (26, 28) for the hosepipe system (2), of which one (26) leads to one of the support holders (25) and the other (28) into the interior (27) of the treatment chamber (3) surrounding the support (23) or to the other end of the support (23).

11. An installation according to claim 10, characterised in that the treatment chamber (3) is rockable over a full circle about its longitudinal centre-line.

12. An installation according to claim 8, characterised by a peristaltic pump (6) having two diametrically opposite rotating pinch rollers (31), between which a closed loop (33) of the hosepipe system (2) is disposed, the length of said loop being greater than the arc of half the circular trajectory (32) produced by the rollers (31).

13. An installation according to claim 12, characterised in that the chord between the associated hose entry (39, 41) to and exit (40, 42) from the circular trajectory (32) is larger for the loop (33) than for the hosepipes.

## Revendications

1. Procédé de mise en condition de substrats de matière synthétique revêtus de cellules vivantes, qui sont destinés à remplacer une paroi d'organe ou de vaisseau dans le corps humain et qui ont été revêtus d'un côté de cellules épithéliales à partir d'une suspension aqueuse, caractérisé en ce que le côté des substrats (23) revêtu de cellules est exposé à un flux pulsé d'une solution nutritive (7) pour les cellules, flux dont les pulsations quasi physiologiques provoquent des inversions brèves de son sens, la solution nutritive (7) après le revêtement étant tout d'abord renouvelée pendant quelques heures à intervalles de temps réguliers, par des impulsions de flux au voisinage du côté des substrats (23) qui est revêtu de cellules et ensuite, la vitesse du flux et celle des pulsations sont augmentées au cours de plusieurs jours d'une valeur nulle à une valeur maximale qui est comparable aux valeurs physiologiques de vaisseaux naturels.

2. Procédé selon la revendication 1, caractérisé en ce que la mise en condition du substrat (23) est effectuée sous une pression statique de 0,07 à 0,2 bar (50 à 150 mm de Hg).

3. Procédé selon la revendication 1 ou 2, caractérisé en ce que la solution nutritive (7) est amenée à suivre, au cours de la mise en condition du substrat (23), un circuit dans lequel elle passe par un échangeur de gaz (5).

4. Procédé selon la revendication 3, caractérisé en ce que le flux de la solution nutritive passe par un étranglement réglable situé en aval du substrat (23) par rapport au sens de la circulation.

5. Procédé selon la revendication 3 ou 4, caractérisé en ce que des amplitudes dynamiques de pression induites au cours des pulsations dans la solution nutritive (7) sont amorties.

6. Procédé selon l'une des revendications 1 à 5, caractérisé en ce que le gradient maximal de la pression dynamique se créant parallèlement au flux est limité à des valeurs inférieures à environ 2,6 bars/sec (2000 mm de Hg/sec).

7. Procédé selon l'une des revendications 1 à 6, caractérisé en ce que le substrat (23) revêtu de cellules est amené à effectuer une oscillation alternative suivant un cercle entier autour d'un axe parallèle à son axe de symétrie longitudinal pendant la mise en condition.

8. Installation pour la mise en oeuvre du procédé selon l'une des revendications 1 à 7, caractérisée par un système de tuyaux souples (2) fermé sur lui-même, rempli de solution nutritive (7) et dans lequel se trouvent au moins une pompe (6), un échangeur de gaz (5), une chambre (3) de traitement du substrat (23) devant être mis en condition, un organe réglable d'étranglement (4) et un réservoir d'air (8).

9. Installation selon la revendication 8, caractérisée en ce que la hauteur statique du point (11) le plus élevé du système de tuyaux (2) par rapport à la chambre de traitement (3) est réglable.

10. Installation selon la revendication 8, caractérisée par une chambre de traitement (3) équipée de supports (25) comportant des trous de passage (24) et destinés au tronçon, devant être mis en condition, d'un substrat tubulaire (23), ainsi que de deux raccords (26, 28) au système de tuyaux souples (2), dont l'un (26) mène à l'un (25) des supports du substrat et l'autre (28) au volume interne (27) de la chambre de traitement (3) qui entoure le substrat (23) ou à l'autre extrémité du substrat (23).

11. Installation selon la revendication 10, caractérisée en ce que la chambre de traitement (3) peut pivoter suivant un cercle entier autour de son axe de symétrie longitudinal.

12. Installation selon la revendication 8, caractérisée par une pompe péristaltique (6) équipée de deux galets d'écrasement (31) diamétralement opposés, exécutant des révolutions et entre lesquels est disposée une boucle fermée (33) du système de tuyaux souples (2), la longueur de cette boucle étant supérieure à l'arc de la demi-trajectoire circulaire (32) suivie par les galets (31).

13. Installation selon la revendication 12, caractérisée en ce que la corde de la boucle (33) est plus longue que celle des tuyaux souples entre chaque entrée (39, 41) du tuyau dans la trajectoire circulaire (32) et sa sortie (40, 42) de cette trajectoire.

Fig. 1

# Fig. 2

Fig. 3

EP 0 320 441 B1